(19) Europäisches Patentamt
European Patent Office
Office européen des brevets

(11) EP 0 782 848 A2

# (12) EUROPÄISCHE PATENTANMELDUNG

(43) Veröffentlichungstag:
**09.07.1997 Patentblatt 1997/28**

(51) Int. Cl.$^6$: **A61K 7/42**, A61K 7/00

(21) Anmeldenummer: **96119400.8**

(22) Anmeldetag: **04.12.1996**

(84) Benannte Vertragsstaaten:
**AT BE CH DE ES FR GB IT LI NL**

(30) Priorität: **21.12.1995 DE 19548014**

(71) Anmelder: **Beiersdorf Aktiengesellschaft**
**D-20245 Hamburg (DE)**

(72) Erfinder:
- **Gers-Barlag, Heinrich, Dr.**
  **25495 Kummerfeld (DE)**
- **Krause, Karl-Heinz**
  **22844 Norderstedt (DE)**

## (54) Kosmetische und dermatologische Lichtschutzformulierungen in Form von Emulsionen

(57) O/W-Makroemulsionen oder O/W-Mikroemulsionen oder O/W/O-Emulsionen oder o/W/O'-Emulsionen mit einem Gehalt Gehalt an in fester, dispergierter Form vorliegenden, in Ölkomponenten schwerlöslichen oder unlöslichen UV-Filtersubstanzen, insbesondere 4, 4',4"-(1,3,5-Triazin-2,4,6-triyltriimino)-tris-benzoesäure-tris(2-ethylhexylester) oder 2-Phenylbenzimidazol-5-sulfonsäure bzw. ihre Salze, erhältlich durch Phaseninversionstechnologie.

EP 0 782 848 A2

**Beschreibung**

Die vorliegende Erfindung betrifft kosmetische und dermatologische Lichtschutzzubereitungen, insbesondere hautpflegende kosmetische und dermatologische Lichtschutzzubereitungen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutze gegen UVB-Strahlung sind zahlreiche Verbindungen bekannt, bei denen es sich zumeist um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubstanzen zur Verfügung zu haben, da auch dessen Strahlen Schäden hervorrufen können. So ist erwiesen, daß UVA-Strahlung zu einer Schädigung der elastischen und kollagenen Fasern des Bindegewebes führt, was die Haut vorzeitig altem läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Die UV-Strahlung kann aber auch zu photochemischen Reaktionen führen, wobei dann die photochemischen Reaktionsprodukte in den Hautmetabolismus eingreifen.

Vorweigend handelt es sich bei solchen photochemischen Reaktionsprodukten um radikalische Verbindungen, z.B. Hydroxylradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Tripletttzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Ein vorteilhafter UVB-Filter ist der 4,4',4"-(1,3,5-Triazin-2,4,6-triyltriimino)-tris-benzoesäure-tris(2-ethylhexylester), synonym: 2,4,6-Tris-[anilino-(p-carbo-2'-ethyl-1'-hexyloxy)]-1,3,5-triazin, Octyltriazon.

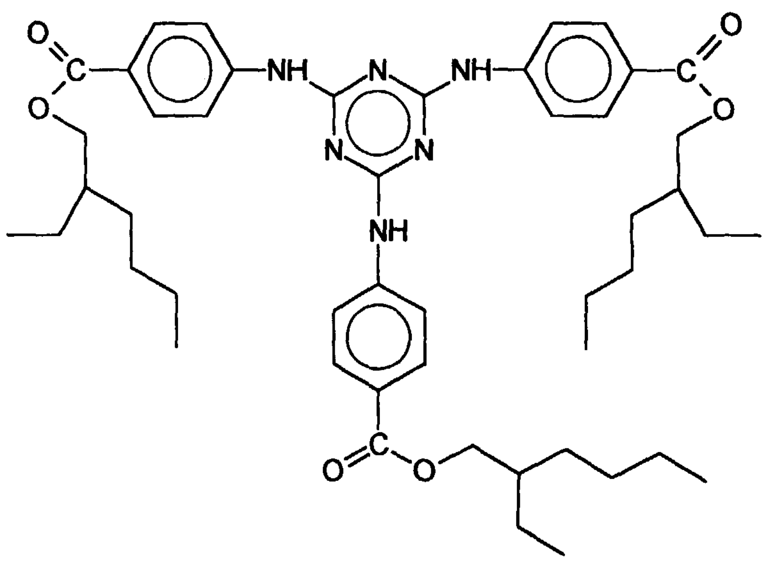

Diese UVB-Filtersubstanz wird beispielsweise von der BASF Aktiengesellschaft unter der Warenbezeichnung UVINUL® T 150 vertrieben und zeichnet sich durch gute UV-Absorptionseigenschaften aus. Das Absorptionsmaximum von Octyltriazon liegt bei 312 nm.

Der Hauptnachteil dieses UVB-Filters ist die schlechte Löslichkeit in Lipiden. Bekannte Lösungsmittel für diesen UVB-Filter können maximal ca. 15 Gew.-% dieses Filters lösen, entsprechend etwa 1 - 1,5 Gew.-% gelöster, und damit nach herkömmlichen Bewertungsmaßstäben: aktiver UV-Filtersubstanz.

Auch andere schwerlösliche UV-Fiitersubstanzen sind bekannt. Zwar gibt es eine gewisse Anzahl wasserlöslicher UV-Filtersubstanzen, auf welche man grundsätzlich und unter bestimmten Bedingungen ausweichen oder welche man zusätzlich verwenden kann. Aber auch solche als wasserlöslich geltenden UV-Filtersubstanzen haben in einer Vielzahl an Fällen den Nachteil, daß sie unter bestimmten Milieubedingungen, insbesondere in Abhängigkeit vom pH-Wert ihre Wasserlöslichkeit zumindest teilweise einbüßen und darüberhinaus auch eher schlechte Öllöslichkeit aufweisen bzw. in Ölkomponenten gänzlich unlöslich sind. Betroffen sind insbesondere sulfonierte UV-Filtersubstanzen, namentlich 2-Phenylbenzimidazol-5-sulfonsäure bzw. ihre Salze, zumal das Natrium-, das Kalium und das TEA-Salz, sofern diese im sauren pH-Bereich vorliegen, beispielsweise erhältlich unter der Bezeichnung Eusolex® 232 der Merck AG, welches sich durch folgende Strukturformel auszeichnet:

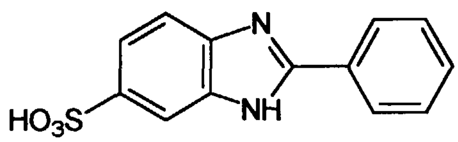

Aber auch beispielsweise Sulfonsäure-Derivate von Benzophenonen, vorzugsweise 2-Hydroxy-4-methoxybenzophenon-5-sulfonsäure und deren Salze und Sulfonsäure-Derivate des 3-Benzylidencamphers, wie z.B. 4-(2-Oxo-3-bornylidenmethyl)benzolsulfonsäure,2-Methyl-5-(2-oxo-3-bornylidenmethyl)sulfonsäure und deren Salze sind erfindungsgemäß vorteilhaft zu verwenden, wenn diese bei einem pH-Wert vorliegt, bei welchem sie weitgehend wasserunlöslich sind.

Selbst wenn grundsätzlich ein gewisser UV-Schutz bei gegebener begrenzter Löslichkeit, (und damit nach herkömmlichen Maßstäben: schlechter Einarbeitbarkeit in eine kosmetische oder dermatologische Zubereitung) erreicht werden kann, kann ein anderes Problem auftreten, die Rekristallisation. Diese tritt gerade bei schlecht löslichen Substanzen vergleichsweise schnell ein, sei es durch Temperaturschwankungen oder andere Einflüsse hervorgerufen. Unkontrollierte Rekristallisation eines wesentlichen Zubereitungsbestandteiles wie eines UV-Filters hat aber äußerst nachteilige Einwirkungen auf die Eigenschaften der gegebenen Zubereitung und, nicht zuletzt, auf den angestrebten Lichtschutz.

Weitverbreitete kosmetische und dermatologische Zubereitungsformen sind Emulsionen. In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. In einer multiplen Emulsion (zweiten Grades) hingegen sind in solchen Tröpfchen feiner disperse Tröpfchen der ersten Phase emulgiert. Auch in diesen Tröpfchen wiederum können noch feiner disperse Tröpfchen vorliegen (multiple Emulsion dritten Grades) und so fort.

Es ist bekannt, daß sich multiple Emulsionen - unter anderem - durch eine besonders feine Emulsionstextur auszeichnen können. Diese Eigenschaft eignet sie hervorragend als Basis sowohl für kosmetische wie für medizinische topische Zubereitungen.

So wie man also bei den einfachen Emulsionen von W/O- oder O/W-Emulsionen spricht (Wasser-in-Oel oder Oel-in-Wasser), gibt es bei multiplen Emulsionen W/O/W-, O/W/O-, O/W/O/W-, W/O/W/O-Emulsionen und so fort.

Multiple Emulsionen, bei welchen die jeweiligen inneren und äußeren Wasserphasen oder inneren und äußeren Ölphasen (wesentlich) unterschiedlich geartet sind (also z.B. W/O/W'- und O/W/O' -Emulsionen), sind der Präparation durch Zweitopfverfahren zugängig. Solche Emulsionen, in welchen die inneren und äußeren Wasser- bzw. Ölphasen nicht unterschiedlich geartet sind, sind sowohl durch Ein- als auch durch Zweitopfverfahren erhältlich.

Die multiplen Emulsionen zweiten Grades werden gelegentlich als "bimultiple Systeme", solche dritten Grades als "trimultiple Systeme" usw., bezeichnet (W.Seifriz, Studies in Emulsions, J.Phys.Chem., 29 (1925) 738 - 749).

Verfahren zur Herstellung multipler Emulsionen sind dem Fachmann an sich geläufig. So gibt es Zweitopfverfahren, in welchen eine einfache Emulsion (z.B. eine W/O-Emulsion) vorgelegt und durch Zugabe einer weiteren Phase (z.B. eine Wasserphase) mit einem entsprechenden Emulgator (z.B. ein O/W-Emulgator) in eine multiple Emulsion (z.B. eine W/O/W- Emulsion) überführt wird.

Eine zweites bekanntes Verfahren besteht darin, Emulgatorgemische mit einer Ölphase und einer Wasserphase in einem Eintopfverfahren in eine multiple W/O/W-Emulsion zu überführen. Die Emulgatoren werden in der Ölphase gelöst und mit der Wasserphase vereinigt. Voraussetzung für ein solches Verfahren ist, daß die HLB-Werte (HLB = Hydrophil-Lipophil-Balance) der eingesetzten einzelnen Emulgatoren sich deutlich voneinander unterscheiden.

Die Definition für den HLB-Wert ist für Polyolfettsäureester gegeben durch die Formel I

$$HLB = 20 * ( 1 - S/A)$$

Für eine Gruppe von Emulgatoren, deren hydrophiler Anteil nur aus Ethylenoxideinheiten besteht, gilt die Formel II

$$HLB = E/5$$

wobei

S = Verseifungszahl des Esters,
A = Säurezahl der zurückgewonnen Säure
E = Massenanteil Ethylenoxid (in %) am Gesamtmolekül

bedeuten.

Emulgatoren mit HLB-Werten von 6-8 sind im allgemeinen W/O-Emulgatoren, solche mit HLB-Werten von 8-18 sind im allgemeinen O/W-Emulgatoren.

Literatur: "Kosmetik - Entwicklung, Herstellung und Anwendung kosmetischer Mittel"; W.Umbach (Hrsg.), Georg Thieme Verlag 1988.

Hydrophile Emulgatoren (mit hohen HLB-Werten) sind in der Regel O/W-Emulgatoren. Demgemäß sind hydrophobe oder lipophile Emulgatoren (mit niedrigen HLB-Werten) in der Regel W/O-Emulgatoren.

Die US-Patentschrift 4,931,210 beschreibt ein Verfahren zur Herstellung von W/O/W-Emulsion, wobei als Emulgatoren Polyglycerinpolyricinoleate verwendet werden.

Die Tröpfchendurchmesser der gewöhnlichen „einfachen", also nichtmultiplen Emulsionen liegen im Bereich von ca 1 µm bis ca. 50 µm. Solche „Makroemulsionen" sind, ohne weitere färbende Zusätze, milchigweißgefärbt und opak. Feinere „Makroemulsionen", deren Tröpfchendurchmesser im Bereich von ca. $10^{-1}$ µm bis ca. 1 µm liegen, sind, wiederum ohne färbende Zusätze, bläulichweißgefärbt und opak. Solche „Makroemulsionen" haben für gewöhnlich hohe Viskosität.

Mizellaren und molekularen Lösungen mit Partikeldurchmessern kleiner als ca. $10^{-2}$ µm, die allerdings nicht mehr als echte Emulsionen aufzufassen sind, ist vorbehalten, klar und transparent zu erscheinen.

Der Tröpfchendurchmesser von Mikroemulsionen dagegen liegt im Bereich von etwa $10^{-2}$ µm bis etwa $10^{-1}$ µm. Mikroemulsionen sind transluzent und meist niedrigviskos. Die Viskosität vieler Mikroemulsionen vom O/W-Typ ist vergleichbar mit der des Wassers.

Vorteil von Mikroemulsionen ist, daß in der dispersen Phase Wirkstoffe wesentlich feiner dispers vorliegen können als in der dispersen Phase von „Makroemulsionen". Ein weiterer Vorteil ist, daß sie aufgrund ihrer niedrigen Viskosität versprühbar sind. Werden Mikroemulsionen als Kosmetika verwendet, zeichnen sich entsprechende Produkte durch hohe kosmetische Eleganz aus.

Es ist bekannt, daß hydrophile Emulgatoren bei steigender Temperatur ihr Löslichkeitsverhalten von wasserlöslich zu fettlöslich ändern. Der Temperaturbereich, in dem die Emulgatoren ihre Löslichkeit geändert haben, wird Phaseninversionstemperaturbereich (PIT) genannt.

S.Matsumoto (Journal of Colloid and Interface Science, Vol. 94, No.2, 1983) beschreibt, daß die Entwicklung einer W/O/W-Emulsion einer Phaseninversion konzentrierter W/O-Emulsionen, welche durch Span 80, einen ausgeprägten W/O-Emulgator, stabilisiert sind, vorausgeht. Matsumoto geht dabei von einem extrem unpolaren Öl, nämlich flüssigem Paraffin aus. Darüberhinaus sei eine gewisse Menge hydrophiler Emulgatoren zur Entwicklung einer W/O/W-Emulsion aus einer W/O-Emulsion nötig.

T.J.Lin, H.Kurihara und H.Ohta (Journal of the Society of Cosmetic Chemists 26, S. 121 - 139, März 1975 zeigen bei unpolaren Ölen, daß im Bereich der PIT extrem instabile multiple Emulsionen vorliegen können.

Aufgabe der vorliegenden Erfindung war daher, diesen Übelständen Abhilfe zu schaffen.

Es hat sich überraschenderweise gezeigt, und darin liegt die Lösung der Aufgaben begründet, daß O/W-Emulsionen, insbesondere O/W-Mikroemulsionen, oder O/W/O-Emulsionen oder O/W/O'-Emulsionen, enthaltend

- eine Wasserphase,
- gegebenenfalls übliche, in Wasser lösliche oder dispergierbare Substanzen,
- eine Ölphase,
  - in welcher mindestens eine in Ölkomponenten an sich schwerlösliche UV-Filtersubstanz,
    - beispielsweise gewählt aus der Gruppe 4,4',4"-(1,3,5-Triazin-2,4,6-triyltriimino)-tris-benzoesäure-tris(2-ethylhexylester), 2-Phenylbenzimidazol-5-sulfonsäure bzw. deren schwerlösliche Salze

    in suspendierter Form vorliegt,
- mindestens einen Emulgator (Emulgator A), gewählt aus der Gruppe der Emulgatoren mit folgenden Eigenschaften

- ihre Lipophilie ist entweder abhängig vom pH-Wert, dergestalt daß durch Erhöhung oder Senkung des pH-Wertes die Lipophilie zunimmt oder abnimmt, wobei unerheblich ist, welche der beiden Möglichkeiten der Änderung der Lipophilie durch die Erhöhung bzw. Senkung des pH-Wertes bewirkt wird, und/oder
- ihre Lipophilie ist abhängig von der Temperatur, dergestalt, daß die Lipophilie mit steigender Temperatur zunimmt und deren Hydrophilie mit sinkender Temperatur zunimmt,

- ferner gegebenenfalls weitere in der Ölphase lösliche oder dispergierbare Substanzen, darunter bevorzugt solche, gewählt aus der Gruppe der nicht unter die Definition des Emulgators A fallende Emulgatoren, insbesondere solche, die vorwiegend als W/O-Emulgatoren wirken,

den Nachteilen des Standes der Technik abhelfen.

Erfindungsgemäß ist möglich, hohe Einsatzkonzentrationen in Ölkomponenten an sich schwerlöslicher UV-Filtersubstanzen, insbesondere des 4,4',4''-(1,3,5-Triazin-2,4,6-triyl-triimino)-tris-benzoesäure-tris(2-ethylhexylesters) und/oder die 2-Phenylbenzimidazol-5-sulfonsäure bzw. ihre Salze, d.h., typischerweise etwa 5 Gew.%, bezogen auf das Gesamtgewicht einer O/W-Mikroemulsion bzw. einer O/W/O-Emulsion mit einem Ölphasenanteil von etwa 50 Gew.-%, wieder bezogen auf das Gesamtgewicht der Zubereitung, zu erreichen und damit die vorteilhaften Eigenschaften dieses Lichtschutzfilters voll auszunutzen.

Als vorteilhafte Verkörperung der vorliegenden Erfindung wird ebenfalls angesehen ein Verfahren zur Einarbeitung in Ölkomponenten an sich schwerlöslicher UV-Filtersubstanzen, in O/W-Emulsionen, O/W-Mikroemulsionen oder O/W/O-Emulsionen, dadurch gekennzeichnet, daß

- die Bestandteile einer Wasserphase,
- gegebenenfalls übliche, in Wasser lösliche oder dispergierbare Substanzen,
- die Bestandteile einer Ölphase
- mindestens ein Emulgator (Emulgator A), gewählt aus der Gruppe der Emulgatoren mit folgenden Eigenschaften

  - ihre Lipophilie ist entweder abhängig vom pH-Wert, dergestalt daß durch Erhöhung oder Senkung des pH-Wertes die Lipophilie zunimmt oder abnimmt, wobei unerheblich ist, welche der beiden Möglichkeiten der Änderung der Lipophilie durch die Erhöhung bzw. Senkung des pH-Wertes bewirkt wird, und/oder
  - ihre Lipophilie ist abhängig von der Temperatur, dergestalt, daß die Lipophilie mit steigender Temperatur zunimmt und die Hydrophilie mit sinkender Temperatur zunimmt,

- in Ölkomponenten schwerlösliche UV-Filtersubstanzen, beispielsweise gewählt aus der Gruppe 4,4',4''-(1,3,5-Triazin-2,4,6-triyltriimino)-tris-benzoesäure-tris (2-ethylhexylester), 2-Phenylbenzimidazol-5-sulfonsäure bzw. deren Salze,
- ferner gegebenenfalls weitere in der Ölphase lösliche oder dispergierbare Substanzen, darunter bevorzugt solche, gewählt aus der Gruppe der nicht unter die Definition des Emulgators A fallende Emulgatoren, insbesondere solche, die vorwiegend als W/O-Emulgatoren wirken,

zusammengegeben werden und unter Agitation ein Gemisch gebildet wird, dergestalt, daß

- dieses Gemisch durch geeignete Wahl der Parameter, gewählt aus der Gruppe pH-Wert, Temperatur und der Konzentration bzw. Konzentrationen mindestens eines der gewählten Emulgatoren, in den Phaseninversionsbereich gebracht wird, in welchem sich W/O-Emulsionen in O/W-Emulsionen umwandeln,
- durch Variation mindestens eines Parameters, gewählt aus der Gruppe pH-Wert, Temperatur und der Konzentration bzw. Konzentrationen mindestens eines der gewählten Emulgatoren, die gebildete W/O-Emulsion aus dem Phaseninversionsbereich heraus gebracht wird, in welchem sich eine gebildete W/O-Emulsion in eine O/W-Emulsion umwandelt, wodurch eine O/W-Emulsion oder O/W-Mikroemulsion erzeugt wird
- gegebenenfalls durch geeignete Wahl der Rahmenbedingungen eine weitere Phaseninversion zur O/W/O-Emulsion eingeleitet wird,
- das Gemisch gegebenenfalls weiteren Aufbereitungsschritten, insbesondere einem oder mehreren Homogenisierungsschritten unterworfen wird.

Erfindungsgemäß gleichermaßen vorteilhaft sind Verfahren, bei welchen die Variation des Parameters oder der Parameter darin besteht, daß

(a) bei vorgegebenem pH-Wert und vorgegebener Konzentration des Emulgators A bzw. der Vielzahl an Emulgatoren A die Temperatur des Gemisches sowie gegebenenfalls zusätzlich die Konzentration mindestens eines weiteren Emulgators A variiert wird, daß

(b) bei vorgegebener Temperatur und vorgegebener Konzentration des Emulgators A bzw. der Vielzahl an Emulgatoren A der pH-Wert des Gemisches sowie gegebenenfalls zusätzlich die Konzentration mindestens eines weiteren Emulgators A variiert wird, daß

(c) bei vorgegebener Temperatur und vorgegebenem pH-Wert die Konzentration mindestens eines Emulgators A sowie gegebenenfalls zusätzlich die Konzentration mindestens eines weiteren Emulgators A variiert wird, daß

(d) bei vorgegebenem pH-Wert die Temperatur des Gemisches sowie zusätzlich die Konzentration mindestens eines Emulgators A sowie gegebenenfalls zusätzlich die Konzentration mindestens eines weiteren Emulgators A variiert werden, daß

(e) bei vorgegebener Temperatur des Gemisches der pH-Wert des Gemisches sowie zusätzlich die Konzentration mindestens eines Emulgators A sowie gegebenenfalls zusätzlich die Konzentration mindestens eines weiteren Emulgators A variiert werden, daß

(f) bei vorgegebener Konzentration mindestens eines Emulgators A der pH-Wert sowie zusätzlich die Temperatur des Gemisches sowie gegebenenfalls zusätzlich die Konzentration mindestens eines weiteren Emulgators A variiert werden.

Grundsätzlich ist zwar möglich und vorteilhaft, die in Ölkomponenten schwerlöslichen UV-Filtersubstanzen zu jedem beliebigen Zeitpunkt dem den Emulsionen zugrundeliegenden Gemisch zuzugeben. Es wird allerdings ein erfindungsgemäßes Verfahren bevorzugt, welches dadurch gekennzeichnet ist, daß die in Ölkomponenten an sich schwerlösliche UV-Filtersubstanz oder -substanzen vor dem Emulgierungsvorgang in der Ölphase gegebenenfalls unter Erwärmen, dispergiert werden und die übrigen Schritte des Verfahrens nachgeschaltet werden.

Es ist bevorzugt, den 4,4',4''-(1,3,5-Triazin-2,4,6-triyltriimino)-tris-benzoesäure-tris(2-ethylhexylester) unter Erwärmen in der Ölphase, insbesondere vorteilhaft in einer unpolaren Ölkomponente bzw. einem Gemisch aus unpolaren Ölkomponenten, zu dispergieren.

Ist der schwerlösliche UV-Filter die 2-Phenylbenzimidazol-5-sulfonsäure bzw. ein (bei höheren pH-Werten an sich durchaus wasserlösliches) Salz, insbesondere ein Alkali-, oder Ammonium- oder ein Aminsalz davon, so ist von Vorteil, den pH-Wert der Wasserphase kleiner als 6, insbesondere kleiner als 5,5 zu wählen und die 2-Phenylbenzimidazol-5-sulfonsäure bzw. deren Salze unter Erwärmen in der Ölphase, zu dispergieren, wobei polare und unpolare Ölkomponenten gleichermaßen vorteilhaft sind.

Es kann auch von Vorteil sein, andere schwerlösliche Salze der 2-Phenylbenzimidazol-5-sulfonsäure zu wählen, beispielsweise mit $Al^{3+}$ als Gegenion, welche eine vom pH-Wert weitgehend unabhängige Löslichkeit aufweisen. In solchen Fällen ist dem pH-Wert im wesentlichen nur im Sinne der erfindungsgemäß einsetzenden Phaseninversionstechnologie Rechnung zu tragen.

Sofern die Phaseninversion im wesentlichen durch Variation der Temperatur eingeleitet wird, sind O/W-Emulsionen, insbesondere O/W-Mikroemulsionen erhältlich, wobei die Größe der Öltröpfchen im wesentlichen durch die Konzentration des oder der eingesetzten Emulgatoren bestimmt wird, dergestalt, daß eine höhere Emulgatorkonzentration kleinere Tröpfchen bewirkt und geringere Emulgatorkonzentration zu größeren Tröpfchen führt. Es ist, wenn die Phaseninversion im wesentlichen durch Variation der Temperatur eingeleitet wird, durchaus vorteilhaft, auf weitere, nicht unter die Definition des Emulgators A fallende, weitere Emulgatoren, namentlich W/O-Emulgatoren, zu verzichten.

Sofern die Phaseninversion im wesentlichen durch Variation des pH-Wertes eingeleitet wird, sind O/W-Emulsionen, insbesondere O/W-Mikroemulsionen, aber auch O/W/O-Emulsionen erhältlich. Es ist, wenn die Phaseninversion im wesentlichen durch Variation des pH-Wertes eingeleitet wird, durchaus vorteilhaft, einen oder mehrere weitere, nicht unter die Definition des Emulgators A fallende, weitere Emulgatoren, namentlich W/O-Emulgatoren, einzusetzen.

Erfindungsgemäß können O/W/O-Emulsionen erhalten werden, wenn der Ölphasenanteil höher als etwa 15 Gew.-%, insbesondere höher als etwa 20 Gew.-%, bezogen auf das Gesamtgewicht der Zubereitung liegt, mehr als etwa 5 Gew.-%, insbesondere etwa 5 - 10 Gew.-% eines zusätzlichen, nicht unter die Definition des Emulgators A fallenden W/O-Emulgators vorliegen und/oder wenn die Ölphase einen niedrigen Anteil an polaren Ölen aufweist.

Erfindungsgemäß können O/W-Mikroemulsionen erhalten werden, wenn der Ölphasenanteil unter etwa 20 Gew.-%, insbesondere unter etwa 15 Gew.-%, bezogen auf das Gesamtgewicht der Zubereitung liegt, weniger als etwa 5 Gew.-% eines zusätzlichen, nicht unter die Definition des Emulgators A fallenden W/O-Emulgators vorliegen und/oder wenn die Ölphase einen hohen Anteil an polaren Ölen aufweist.

Erfindungsgemäß können O/W-Emulsionen („Makroemulsionen") erhalten werden, wenn weniger als etwa 5 Gew.-% eines zusätzlichen nicht unter die Definition des Emulgators A fallenden W/O-Emulgators und mehr als etwa 20 Gew.-% einer polaren Ölphase vorliegen. Vorteilhaft können zusätzliche Gelbildner (z.B. Carbopole, Xanthangummi, Cellulosederivate) eingesetzt werden.

Es ist dabei im Einzelfalle möglich, daß die vorgenannten Konzentrationsgrenzen leicht über- oder unterschritten werden und dennoch die betreffenden Emulsionstypen erhalten werden. Dies kommt angesichts der breit streuenden Vielfalt an geeigneten Emulgatoren und Ölbestandteilen für den Fachmann nicht unerwartet, so daß er weiß, daß bei solchen Über- oder Unterschreitungen der Boden der vorliegenden Erfindung nicht verlassen wird.

Es hat sich in erstaunlicher Weise herausgestellt, daß der oder die erfindungsgemäß eingesetzten schwerlöslichen

UV-Filtersubstanzen als Festkörper, und zwar gewissermaßen „verkapselt“, nämlich abgetrennt von anderen Bestandteilen der Zubereitungen vorliegen, in welchen sie teilweise sogar begrenzte Löslichkeit besitzen können. Es wird angenommen, daß die Festkörperpartikel der schwerlöslichen UV-Filtersubstanzen durch das erfindungsgemäße Einarbeitungsverfahren einen Hüllfilm erhalten, welcher vermutlich als wesentlichen Bestandteil Emulgatormolekeln enthält.

Erfindungsgemäß kann die Rekristallisation unlöslicher oder schwerlöslicher UV-Filtersubstanzen verhindert werden. Darüberhinaus sind erfindungsgemäß Lichtschutzzubereitungen erhältlich, welche vorzügliche Anwendungseigenschaften aufweisen.

Der Phaseninversionsbereich läßt sich mathematisch darstellen als Punktmenge innerhalb des geradlinigen Koordinatensystems $\Sigma$, welches durch die Größen Temperatur, pH-Wert und Konzentration eines geeigneten Emulgators bzw. eines Emulgatorengemisches in der Zubereitung gebildet wird, gemäß:

$$\Sigma = \{O, \theta, a, m\},$$

mit

O - Koordinatenursprung
θ - Temperatur
a - pH-Wert
m - Konzentration

Dabei muß genaugenommen natürlich in einem mehrkomponentigen Emulgatorensystem der Beitrag $m_i$ jedes einzelnen Emulgators zur Gesamtfunktion berücksichtigt werden, was bei einem i-komponentigen Emulgatorensystem zur Beziehung

$$\Sigma = \{O, \theta, a, m_1, m_2, ..., m_i\} \text{ führt.}$$

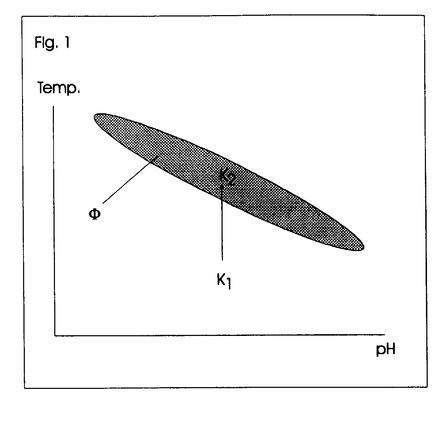

Der Phaseninversionsbereich $\Phi$ stellt dabei im mathematischen Sinne ein zusammenhängendes Gebiet oder eine Vielzahl zusammenhängender Gebiete innerhalb des Koordinatensystems $\Sigma$ dar. $\Phi$ repräsentiert die Gesamtmenge der Koordinatenpunkte $K(\theta, a, m_1, m_2, ..., m_i)$, welche erfindungsgemäße Gemische aus Wasser- und Ölphase, i erfindungsgemäßen Emulgatoren der Konzentration $m_i$ bei der Temperatur $\theta$ und dem pH-Wert a bestimmen, und für welche gilt, daß beim Übergang von einer Koordinate $K_1 \notin \Phi$ zu einer Koordinate $K_2 \in \Phi$ Phaseninversion eintritt, wie in Fig. 1 beschrieben. Als variable Koordinaten in Fig.1 wurden Temperatur und pH-Wert eines gegebenen Gemisches unter Konstanthaltung der Konzentration von $m_1$ - $m_i$ angegeben. Beim Übergang von $K_1$ nach $K_2$ wird lediglich die Temperatur erhöht.

Unter den erfindungsgemäßen Bedingungen ist dieser Prozeß nicht notwendig reversibel, d.h., kehrt das System von der Koordinate $K_2 \in \Phi$ wieder zur Koordinate $K_1 \notin \Phi$ zurück, können erfindungsgemäß andere Emulsionstypen erhalten werden, als der Stand der Technik hätte erwarten lassen. Beispielsweise wird durch Erhöhen der Temperatur eines erfindungsgemäßen Gemisches aus Wasser- und Ölphase, i erfindungsgemäßen Emulgatoren der Konzentrationen $m_i$, wobei der pH-Wert a konstant bleibt, ausgehend von einer Temperatur welche für Phaseninversion zu niedrig ist (Bedingungen also, wo eine herkömmliche O/W-Emulsion vorläge, welche auch beim Abkühlen auf Raumtemperatur eine solche bliebe), erwärmt wird, so daß Phaseninversion eintritt, wird nach Abkühlen, z.B. auf Raumtemperatur, keine herkömmliche O/W-Emulsion erhalten, sondern eine erfindungsgemäße O/W-Mikroemulsion. Oder, mutatis mutandis: eine erfindungsgemäße O/W/O-Emulsion.

Unerheblich ist dabei, ob der Phaseninversionsbereich eines gegebenen Systems ein einziges zusammenhängendes (i + 2)-dimensionales Gebiet darstellt oder aus mehreren zusammenhängenden, aber voneinander getrennten solchen Gebieten besteht, also mehreren Phaseninversionsbereichen eines gegebenen Systems entsprechend. Im Rahmen der hiermit vorgelegten Offenbatung wird daher stets verallgemeinernd von einem Phaseninversionsbereich gesprochen, auch bei Vorliegen zweier oder mehrerer voneinander getrennter solcher Bereiche.

Die Praxis der Herstellung einer erfindungsgemäßen Emulsion besteht vorteilhaft darin, nach Auswahl geeigneter Rohstoffe, d.h., Wasser- und Ölphase, ein oder mehrere Emulgatoren vom Typ A, letzterer oder letztem vorliegend in Konzentrationen, bei weichen Phaseninversion für das gegebene Gemisch möglich ist, und gegebenenfalls weitere Substanzen, die Einzelkomponenten unter Agitation auf eine Temperatur zu erwärmen, bei welcher Phaseninversion für das gegebene Gemisch möglich ist, und durch Erhöhung oder Erniedrigung des pH-Wertes des Gemisches Phaseninversion herbeizuführen, hernach unter fortwährender Agitation das Gemisch auf Raumtemperatur abkühlen zu lassen. Ein oder mehrere zwischengeschaltete Homogenisierungsschritte sind vorteilhaft, aber nicht zwingend notwendig.

Eine weitere vorteilhafte Ausführungsform des erfindungsgemäßen Verfahrens besteht darin, nach der Auswahl geeigneter Rohstoffe, d.h., Wasser- und Ölphase, ein oder mehrere Emulgatoren vom Typ A letzterer oder letztere vorliegend in Konzentrationen, bei welchen Phaseninversion für das gegebene Gemisch möglich ist, und gegebenenfalls weitere Substanzen, die Einzelkomponenten unter Agitation auf einen pH-Wert zu bringen, bei welchem Phaseninversion für das gegebene Gemisch möglich ist, und durch Erhöhung der Temperatur des Gemisches Phaseninversion herbeizuführen, hernach unter fortwährender Agitation das Gemisch auf Raumtemperatur abkühlen zu lassen. Ein oder mehrere zwischengeschaltete Homogenisierungsschritte sind vorteilhaft, aber nicht zwingend notwendig.

Eine dritte vorteilhafte Ausführungsform des erfindungsgemäßen Verfahrens besteht darin, nach der Auswahl geeigneter Rohstoffe, d.h., Wasser- und Ölphase, ein oder mehrere Emulgatoren vom Typ A und gegebenenfalls weitere Substanzen, die Einzelkomponenten unter Agitation auf einen pH-Wert und eine Temperatur zu bringen, bei welchen Phaseninversion für das gegebene Gemisch möglich ist, und durch Zugabe des Emulgators A oder der Emulgatoren A zum Gemisch Phaseninversion herbeizuführen, hernach unter fortwährender Agitation das Gemisch auf Raumtemperatur abkühlen zu lassen. Ein oder mehrere zwischengeschaltete Homogenisierungsschritte sind vorteilhaft, aber nicht zwingend notwendig.

In der Praxis ist möglich und gegebenenfalls sogar vorteilhaft, bei der Herstellung einer erfindungsgemäßen Emulsion den Temperaturbereich, der dem Phaseninversionsbereich zugeordnet werden kann auch zu übersteigen, da beim Abkühlen auf Raumtemperatur dieser Bereich dann zwangsläufig durchlaufen wird.

Die Emulgatoren A werden bevorzugt gewählt aus der Gruppe der Emulgatoren, welche gute Protonendonoren bzw. Protonenakzeptoren darstellen, wobei gewährleistet sein muß, daß ihre Lipophilie entweder abhängig ist vom pH-Wert, dergestalt daß durch Erhöhung oder Senkung des pH-Wertes die Lipophilie zunimmt oder abnimmt, wobei unerheblich ist, welche der beiden Möglichkeiten der Änderung der Lipophilie durch die Erhöhung bzw. Senkung des pH-Wertes bewirkt wird, oder ihre Lipophilie abhängig ist von der Temperatur, dergestalt, daß die Lipophilie mit steigender Temperatur zunimmt und deren Hydrophilie mit sinkender Temperatur zunimmt, oder ihre Lipophilie abhängig ist von pH-Wert und der Temperatur, dergestalt daß durch Erhöhung oder Senkung des pH-Wertes die Lipophilie zunimmt oder abnimmt, wobei unerheblich ist, welche der beiden Möglichkeiten der Änderung der Lipophilie durch die Erhöhung bzw. Senkung des pH-Wertes bewirkt wird, und daß die Lipophilie mit steigender Temperatur zunimmt und deren Hydrophilie mit sinkender Temperatur zunimmt.

Vorteilhaft werden die Emulgatoren des Typs A gewählt aus der Gruppe der Sorbitanester und Saccharoseester, insbesondere der verzweigten und unverzweigten Alkylester und Alkenylester mit Kohlenstoffketten von 4 - 24 Kohlenstoffatomen, bevorzugt Sorbitanstearat, Sorbitanoleat, Glycerylsorbitanstearat, Saccharosemonostearat, Saccharosemonolaurat, Saccharosepalmitat.

Vorteilhaft können die Emulgatoren des Typs A gewählt aus der Gruppe der Monoglycerin-monocarbonsäuremonoester gewählt werden, insbesondere solche, welche durch die Strukturen

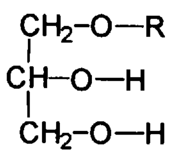

bzw.

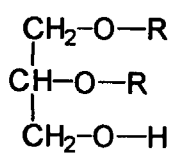

gekennzeichnet sind, wobei R einen verzweigten oder unverzweigten Acylrest mit 6 - 14 Kohlenstoffatomen darstellt. Vorteilhaft wird R gewählt aus der Gruppe der unverzweigten Acylreste. Die diesen Estern zugrundeliegenden Fettsäuren bzw. Monocarbonsäuren sind die

| | | |
|---|---|---|
| Hexansäure | (Capronsäure) | (R = -C(O)-$C_5H_{11}$), |
| Heptansäure | (Önanthsäure) | (R = -C(O)-$C_6H_{13}$), |
| Octansäure | (Caprylsäure) | (R = -C(O)-$C_7H_{15}$), |
| Nonansäure | (Pelargonsäure) | (R = -C(O)-$C_8H_{17}$), |
| Decansäure | (Caprinsäure) | (R = -C(O)-$C_9H_{19}$), |
| Undecansäure | | (R = -C(O)-$C_{10}H_{21}$), |
| Dodecansäure | (Laurinsäure) | (R = -C(O)-$C_{11}H_{23}$), |
| Tridecansäure | | (R = -C(O)-$C_{12}H_{25}$), |
| Tetradecan-säure | (Myristinsäure) | (R = -C(O)-$C_{13}H_{27}$). |

Besonders vorteilhaft stellt R den Octanoylrest (Caprylsäurerest) bzw. den Decanoylrest (Caprinsäurerest) dar, wird also also durch die Formeln

$$R = -C(O)-C_7H_{15})$$

bzw.

$$R = -C(O)-C_9H_{19}$$

repräsentiert.

Vorteilhaft können die Emulgatoren des Typs A auch aus der Gruppe der Di- und Triglycerin-monocarbonsäuremonoester gewählt werden. Erfindungsgemäß liegen die Di- bzw. Triglycerineinheiten der erfindungsgemäßen Diglycerin-monocarbonsäuremonoester bzw. Triglycerln-rnonocarbonsäure-monoester als lineare, unverzweigte Moleküle, also über die jeweiligen OH-Gruppen in 1- bzw. 3-Stellung veretherte "Monoglycerinmoleküle" vor.

Ein geringer Anteil zyklischer Di- bzw. Triglycerineinheiten sowie über die OH-Gruppen in 2-Stellung veretherte Glycerinmoleküle kann geduldet werden. Es ist jedoch von Vorteil, solche Verunreinigungen so gering wie nur möglich zu halten.

Die erfindungsgemäßen Monocarbonsäuremonoester sind bevorzugt durch folgende Struktur gekennzeichnet:

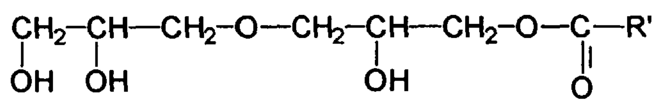

wobei R' einen Kohlenwasserstoffrest, vorteilhaft einen verzweigten oder unverzweigten Alkyl- oder Alkenylrest von 5 bis 17 C-Atomen darstellt.

Die erfindungsgemäßen Monocarbonsäureester des Triglycerins sind bevorzugt durch folgende Struktur gekennzeichnet:

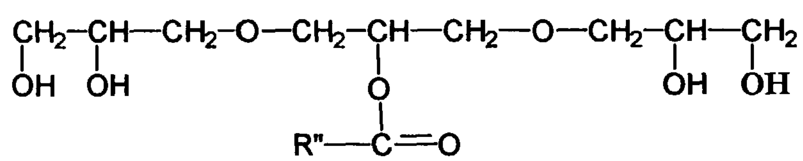

wobei R" einen Kohlenwasserstoffrest, vorteilhaft einen verzweigten oder unverzweigten Alkyl- oder Alkenylrest Alkylrest von 5 bis 17 C-Atomen darstellt.

Die diesen Estern zugrundeliegenden Säuren sind die

| | | |
|---|---|---|
| Hexansäure | (Capronsäure) | (R' bzw. R"= $-C_5H_{11}$), |
| Heptansäure | (Önanthsäure) | (R' bzw. R"= $-C_6H_{13}$), |
| Octansäure | (Caprylsäure) | (R' bzw. R"= $-C_7H_{15}$), |
| Nonansäure | (Pelargonsäure) | (R' bzw. R"= $-C_8H_{17}$), |
| Decansäure | (Caprinsäure) | (R' bzw. R"= $-C_9H_{19}$), |
| Undecansäure | | (R' bzw. R"= $-C_{10}H_{21}$), |
| 10-Undecensäure | (Undecylensäure) | (R' bzw. R"= $-C_{10}H_{19}$), |
| Dodecansäure | (Laurinsäure) | (R' bzw. R"= $-C_{11}H_{23}$), |
| Tridecansäure | | (R' bzw. R"= $-C_{12}H_{25}$), |
| Tetradecansäure | (Myristinsäure) | (R' bzw. R"= $-C_{13}H_{27}$), |
| Pentadecansäure | | (R' bzw. R"= $-C_{14}$H29), |
| Hexadecansäure | (Palmitinsäure) | (R' bzw. R"= $-C_{15}H_{31}$), |
| Heptadecansäure | (Margarinsäure) | (R' bzw. R"= $-C_{16}H_{33}$), |
| Octadecansäure | (Stearinsäure) | (R' bzw. R"= $-C_{17}H_{35}$). |

Besonders günstig werden R' und R" gewählt aus der Gruppe der unverzweigten Alkylreste mit ungeraden C-Zahlen, insbesondere mit 9, 11 und 13 C-Atomen.

Im allgemeinen sind die Monocarbonsäure-monoester des Diglycerins denen des Triglycerins bevorzugt.

Erfindungsgemäß ganz besonders günstig sind

| | | |
|---|---|---|
| Diglycerinmonocaprinat | (DMC) | R' = 9 |
| Triglycerinmonolaurat | (TML) | R" = 11 |
| Diglycerinmonolaurat | (DML) | R' = 11 |
| Triglycerinmonomyristat | (TMM) | R" = 13 |

Als bevorzugter erfindungsgemäßer Monocarbonsäuremonoester des Diglycerins hat sich das Diglycerinmonocaprinat (DMC) erwiesen.

Nach einer vorteilhaften Ausführungsform der vorliegenden Erfindung wird ein zusätzlicher Anteil an in anderen Stellen verestertem Di- oder Triglycerin, ebenso wie gegebenenfalls ein Anteil an den verschiedenen Diestern des Di- bzw. Triglycerins verwendet.

Vorteilhaft sind auch Triglyceryldiisostearat (Nomenklatur analog CTFA: Polyglyceryl-3-diisostearat), Isostearyldiglycerylsuccinat, Diglycerylsesquiisostearat (Nomenklatur analog CTFA: Polyglyceryl-2-sesquiisostearat), Triglycerylpolyhydroxystearat (Nomenklatur analog CTFA: Polyglyceryl-2-polyhydroxystearat).

Auch Cetearylisononanoat, Dicccoyl-pentaerythrityl-distearylcitrat, ferner die Methiconcopolyole, Cyclomethiooncopolyle, Alkylmethiconcopolyole, insbesondere Laurylmethiconcopolyol, Cetyldimethiconcopolyol, haben sich erfindungsgemäß als vorteilhaft erwiesen.

Besonders vorteilhaft werden der oder die Emulgatoren des Typs A gewählt aus der Gruppe der verzweigten oder unverzweigten Alkylmonocarbonsäuren, Alkenylmonocarbonsäuren und Alkylendicärbonsäuren mit 4 bis 30 Kohlenstoffatomen, insbesondere Stearinsäure, Ölsäure, Bernsteinsäure, Hexansäure (Capronsäure), Heptansäure (Önanthsäure), Octansäure (Caprylsäure), Nonansäure (Pelargonsäure), Decansäure (Caprinsäure), Undecansäure, Undecensäure (Undecylensäure), Dodecansäure (Laurinsäure), Tridecansäure, Tetradecansäure (Myristinsäure), Pentadecansäure, Hexadecansäure (Palmitinsäure), Heptadecansäure (Margarinsäure), Octadecansäure (Stearinsäure), Isostearinsäure, Behensäure. Es ist auch vorteilhaft, die Emulgatoren A aus der Gruppe der kosmetisch bzw. pharmazeutisch akzeptablen Salze der vorstehend genannten Carbonsäuren zu wählen, insbesondere der Alkali-, Ammonium-, Monoalkylammonium, Dialkylammonium-, Trialkylammonium- und Tetraalkylammoniumsalze.

Ebenfalls besonders vorteilhaft werden der oder die Emulagtoren A gewählt aus der Gruppe der mono-, oligo und polyethoxylierten Verbindungen, insbesondere der polyethoxylierten ein- oder mehrbasigen Alkohole oder Fettsäuren, beispielsweise Ceteareth-20, PEG-20-Glycerylstearat, Steareth-20, PEG-20-Stearat, PEG-30-Stearat, PEG-40-Rizinusöl, PEG-1-Glycerin-Sorbitan-Oleostearat, PEG-7 hydriertes Rizinusöl, PEG-40-Sorbitanperoleat, PEG-45-Dodecyl-Glycol-Copolymer.

Die erfindungsgemäßen Emulsionen sind vorteilhaft dadurch gekennzeichnet, daß der Emulgator A oder die Emulgatoren A in Konzentrationen von 0,01 - 20 Gew.-%, bevorzugt 0,05 - 10 Gew.-%, besonders bevorzugt 0,1 - 5 Gew.-%, jeweils bezogen auf das Gesamtgewicht der Zusammensetzung, vorliegt oder vorliegen.

Es ist erfindungsgemäß möglich, die Einsatzmengen von an sich in Ölkomponenten schwerlöslichen oder unlöslichen UV-FIltern, insbesondere 4,4',4"-(1,3,5-Triazin-2,4,6-trlyltriimino)-tris-benzoesäure-tris(2-ethylhexylester), aber auch 2-Phenylbenzimidazol-5-sulfonsäure bzw. deren Salze in kosmetischen oder dermatologischen Zubereitungen gegenüber dem Stande der Technik zu vervielfachen.

Die Gesamtmenge an in Ölkomponenten an sich schwerlöslichen UV-Filtersubstanzen, insbesondere 4,4',4"-(1,3,5-Triazin-2,4,6-triyltriimino)-tris-benzoesäure-tris(2-ethylhexylester), aber auch 2-Phenylbenzimidazol-5-sulfonsäure bzw. deren Salze in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Es ist erfindungsgemäß vorteilhaft, in den erfindungsgemäßen Zubereitungen zusätzliche öllösliche UVA-Filter und/oder UVB-Fitter in der Lipidphase und/oder wasserlösliche UVA-Filter und/oder UVB-Fiter in der wäßrigen Phase einzusetzen.

Vorteilhaft können die erfindungsgemäßen Lichtschutzformulierungen weitere Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen.

Die zusätzlichen UVB-Filter können öllöslich oder wasserlöslich sein. Vorteilhafte öllösliche UVB-Filtersubstanzen sind z.B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 4,4',4"-(1,3,5-Triazin-2,4,6-triyltriimino)-tris-benzoesäure-tris(2-ethylhexylester), für den es durchaus Lösungsmittel, insbesondere polare Ölkomponenten gibt,

Vorteilhafte wasserlösliche UVB-Filtersubstanzen sind z.B.:

- Salze der 2-Phenylbenzimidazol-5-sulfonsäure wie ihr Natrium-, Kalium- oder ihr Triethanolammonium-Salz, sowie die Sulfonsäure selbst, sofern der pH-Wert so gewählt wird, daß diese in wasserlöslicher Form vorliegen,
- Sulfonsäure-Derivate von Benzophenonen, vorzugsweise 2-Hydroxy-4-methoxybenzophenon-5-sulfonsäure und deren Salze, sofern der pH-Wert so gewählt wird, daß diese in wasserlöslicher Form vorliegen,
- Sulfonsäure-Derivate des 3-Benzylidencamphers, wie z.B. 4-(2-Oxo-3-bornylidenmethyl)benzolsulfonsäure,2-Methyl-5-(2-oxo-3-bornylidenmethyl)sulfonsäure und deren Salze, sofern der pH-Wert so gewählt wird, daß diese in wasserlöslicher Form vorliegen,.

Die Liste der genannten UVB-Filter, die in Kombination mit den erfindungsgemäßen Wirkstoffkombinationen verwendet werden können, soll selbstverständlich nicht limitierend sein.

Es kann auch von Vorteil sein, zusätzliche UVA-Filter in den erfindungsgemäßen Zubereitungen einzusetzen, welche bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Erfindungsgemäße kosmetische und dermatologische Zubereitungen enthalten vorteilhaft außerdem anorganische Pigmente auf Basis von Metalloxiden und/oder anderen in Wasser schwerlöslichen oder unlöslichen Metallverbindungen, insbesondere der Oxide des Titans ($TiO_2$), Zinks ($ZnO$), Eisens (z.B. $Fe_2O_3$), Zirkoniums ($ZrO_2$), Siliciums

($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Es ist besonders vorteilhaft im Sinne der vorliegenden Erfindung, wenngleich nicht zwingend, wenn die anorganischen Pigmente in hydrophober Form vorliegen, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

$$n\ TiO_2 + m\ (RO)_3\ Si\text{-}R' \rightarrow n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhatte $TiO_2$-Pigmente sind beispielsweise unter den Handelsbezeichnungen MT 100 T von der Firma TAYCA erhältlich.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phylinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Lipidphase kann vorteilhaft gewählt werden aus folgender Substanzgruppe:

- Öle, wie Triglyceride der Caprin- oder der Caprylsäure
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;

Ganz besonders vorteilhaft kann die Lipidphase gewählt werden aus der Gruppe der Silikonöle wie Dimethylpolysiloxane, Diethylpolysiloxane, Diphenylpolysiloxane sowie Mischformen daraus

Die wäßrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die kosmetischen oder dermatologischen Lichtschutzzubereitungen enthalten vorteilhaft anorganische Pigmente, insbesondere Mikropigmente, z.B. in Mengen von 0,1 Gew.-% bis 30 Gew.-%, vorzugsweise in Mengen von 0,5 Gew.-% bis 10 Gew.-%, insbesondere aber 1 Gew.-% bis 6 Gew.-%, bezogen auf das Gesamtgewicht der Zubereitungen.

Nachfolgend soll kurz noch auf einige Besonderheiten und Unterschiede der Voraussetzungen für erfindungsgemäß O/W-Emulsionen, O/W-Mikroemulsionen bzw O/W/O-Emulsionen eingegangen werden.

Öle und Fette unterscheiden sich unter anderem in ihrer Polarität, welche schwierig zu definieren ist. Es wurde bereits vorgeschlagen, die Grenzflächenspannung gegenüber Wasser als Maß für den Polaritätsindex eines Öls bzw. einer Ölphase anzunehmen. Dabei gilt, daß die Polarität der betreffenden Ölphase umso größer ist, je niedriger die Grenzflächenspannung zwischen dieser Ölphase und Wasser ist. Erfindungsgemäß wird die Grenzflächenspannung als ein mögliches Maß für die Polarität einer gegebenen Ölkomponente angesehen.

Die Grenzflächenspannung ist diejenige Kraft, die an einer gedachten, in der Grenzflache zwischen zwei Phasen befindlichen Linie der Länge von einem Meter wirkt. Die physikalische Einheit für diese Grenzflächenspannung errechnet sich klassisch nach der Beziehung Kraft/Länge und wird gewöhnlich in mN/m (Millinewton geteilt durch Meter) wiedergegeben. Sie hat positives Vorzeichen, wenn sie das Bestreben hat, die Grenzfläche zu verkleinern. Im umgekehrten Falle hat sie negatives Vorzeichen.

Als Grenze, unterhalb derer eine Ölphase als "polar" und oberhalb derer eine Ölphase als "unpolar" gilt, werden erfindungsgemäß 30 mN/m angesehen.

Erfindungsgemäß wird für O/W-Mikroemulsionen die Ölphase vorteilhaft gewählt aus der Gruppe der polaren Ölkomponenten, welche eine Polartät zwischen 10 und 30 mN/m aufweisen, wobei gewährleistet sein muß, daß mindestens eine nichtpolare Ölkomponente vorhanden ist.

Vorteilhafte O/W-Mikroemulsionen werden erhalten, wenn die Ölphase gewählt wird aus der Gruppe der polaren Ölkomponenten, besonders bevorzugt aus der Gruppe der natürlichen, synthetischen oder halbsynthetischen Ölkomponenten, welche eine Polarität zwischen 10 und 20 mN/m aufweisen, wobei gewährleistet sein muß, daß mindestens eine nichtpolare Ölkomponente vorhanden ist.

Vorteilhaft ist auch, polare pflanzliche Öle als polare Öle der erfindungsgemäßen O/W-Emulsionen zu verwenden. Die pflanzlichen Öle können vorteilhaft gewählt werden aus der Gruppe der Öle der Pflanzenfamilien Euphorbiaceae, Poaceae, Fabaceae, Brassicaceae, Pedalaceae, Asteraceae, Linaceae, Flacourticaceae, Violales, vorzugsweise gewählt aus der Gruppe natives Rizinusöl, Weizenkeimöl, Traubenkemöl, Kukuinußöl, Safloröl, Distelöl, Nachtkerzenöl und weiteren Ölen, welche mindestens 1,5 Gew.-% an Linolsäureglyceriden enthalten.

Im Gegenzuge sollen erfindungsgemäße O/W/O-Emulsionen von solchen Ölkomponenten nur untergeordnete Mengen aufweisen und statt deren hauptsächlich solche, deren Polaritätswert höher liegt als 30 mN/m. Als vorteilhaft haben sich gleichermaßen natürliche, synthetische und halbsynthetische Öle, Fette und Wachse erwiesen.

Der Zusatz von Elektrolyten bewirkt eine Veränderung des Löslichkeitsverhaltens eines hydrophilen Emulgators.

Die hydrophilen Emulgatoren mit den vorab beschriebenen Strukturen bzw. Eigenschaften durchlaufen eine partielle Phaseninversion, in der es zu einer Solubilisierung von Wasser durch die Ölphase kommt, welche in einer stabilen Mikroemulsion oder, im gewünschten Falle, auch einer stabilen O/W/O-Emulsion resultiert.

Die erfindungsgemäßen Mikroemulsionen enthalten daher vorteilhaft Elektrolyte, insbesondere eines oder mehrere Salze mit folgenden Anionen: Chloride, ferner anorganische Oxo-Element-Anionen, von diesen insbesondere Sulfate, Carbonate, Phosphate, Borate und Aluminate. Auch auf organischen Anionen basierende Elektrolyte können vorteilhaft verwendet werden, beispielsweise Lactate, Acetate, Benzoate, Propionate, Tartrate, Citrate und andere mehr. Vergleichbare Effekte sind auch durch Ethylendiamintetraessigsäure und deren Salze zu erzielen.

Als Kationen der Salze werden bevorzugt Ammonium,- Alkylammonium,- Alkalimetall-, Erdalkalimetall,- Magnesium-, Eisen- bzw. Zinkionen verwendet. Es bedarf an sich keiner Erwähnung, daß in Kosmetika nur physiologisch unbedenkliche Elektrolyte verwendet werden sollten. Spezielle medizinische Anwendungen der erfindungsgemäßen Mikroemulsionen können andererseits, wenigstens grundsätzlich, die Verwendung von Elektrolyten bedingen, welche nicht ohne ärztliche Aufsicht verwendet werden sollten.

Besonders bevorzugt sind Kaliumchlorid, Kochsalz, Magnesiurnsulfat, Zinksulfat und Mischungen daraus. Ebenfalls vorteilhaft sind Salzmischungen wie sie im natürlichen Salz vom Toten Meer auftreten.

Die Konzentration des Elektrolyten oder der Elektrolyte sollte etwa 0,01 - 10,0 Gew.-%, besonders vorteilhaft etwa 0,03 - 8,0 Gew.% betragen, bezogen auf das Gesamtgewicht der Zubereitung.

Die Emulgatoren des Typs A können landsläufig als O/W-Emulgatoren angesehen werden. Ein Gehalt von etwa 5 - 10 Gew.-% an üblichen W/O-Emulgatoren fördert in vorteilhafter Weise die Bildung von O/W/O-Emulsionen, ein Gehalt von deutlich über 10 Gew.-% an solchen Emulgatoren führt zur Destabilisierung der O/W/O-Emulsionen.

Ferner ist gewünschtenfalls für die Herstellung erfindungsgemäßer O/W/O-Emulsionen von Vorteil, hydrophile und/oder lipophile Gelbildner einzusetzen. Diese tragen zwar in der Regel nicht zur Bildung multipler Tröpfchen bei, fördern aber die Stabilität einmal gebildeter multipler Tröpfchen.

Wenn bei einem erfindungsgemäßen Herstellungsverfahren für O/W/O-Emulsionen der pH-Wert variiert werden soll, um ein ansonsten vorbestimmtes System in den Phaseninversionsbereich zu bringen, so ist von Vorteil, zu Beginn des Verfahrens zunächst möglichst geringe Elektrolytkonzentration in der Wasserphase einzusetzen, möglichst zunächst ganz darauf zu verzichten. Weiterhin ist von Vorteil, den Emulgator A in der Ölphase vorzulegen, beispielsweise für Stearinsäure im Konzentrationsbereich von 0,5 - 5 Gew.-%, insbesondere 2 Gew.-%. Vorteilhaft ist die Gegenwart eines nicht unter die Definition des Emulgators A fallenden Emulgators im Konzentrationsbereich von ca. 5 - 10 Gew.-%, insbesondere etwa 7 Gew.-%.

Die Variation des pH-Wertes sollte vorteilhaft erst erfolgen, wenn die W/O-Emulsion sich gebildet hat, beispielsweise durch Zufügen von NaOH.

Dabei liegt im allgemeinen Fachwissen des Fachmannes und bedarf keinerlei erfinderischen Dazutuns, für einen gegebenen Emulgator oder ein gegebenes Emulgatorsystem in einem gegebenen Wasser/Ölphasensystem den Temperatur- bzw. pH-Bereich zu ermitteln, in welchem Phaseninversion stattfindet. Als allgemeiner Richtwert für den PIT bei üblichen Emulgatorkonzentrationen kann ein Temperaturbereich von etwa 40 - 90° C angegeben werden. Im allgemeinen sinkt der PIT bei steigender Emulgatorkonzentration.

Es können während dieses Verfahrens gewünschtenfalls ferner die in der Kosmetik oder medizinischen Galenik üblichen Grund-, Hilfs-, Zusatz-, und/oder Wirkstoffe zugegeben werden. Es ist dem Fachmann klar, zu welchem Zeitpunkt solche Stoffe dem Prozeß beigegeben werden können, ohne daß die Eigenschaften der zu erzielenden Emulsion wesentlich beeinträchtigt werden.

Die nachfolgenden Beispiele sollen das Wesen der vorliegenden Erfindung näher umreißen, ohne die Erfindung einzuschränken.

**Beispiel 1**

O/W-Mikroemulsion

| | Gew.-% |
|---|---|
| Ceteareth-12 | 8,00 |
| Cetearylisononanoat | 20,00 |
| Cetearylalkohol | 4,00 |
| Eusolex® 232 | 4,80 |
| $MgSO_4$ | 3,00 |
| | pH=5,0 |
| Farbstoffe, Parfum, Konservierungsmittel | q.s. |
| Wasser | ad 100,00 |

Die Bestandteile der Ölphase werden vereinigt und homogenisiert, dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionstemperaturbereich des Systems wieder heraus gebracht).

**Beispiel 2**

O/W-Mikroemulsion

| | Gew.-% |
|---|---|
| Ceteareth-12 | 12,00 |
| Cetearylisononanoat | 20,00 |
| Cetearylalkohol | 6,00 |
| Eusolex® 232 | 4,80 |
| Parsol ® 1789 | 2,00 |
| $MgSO_4$ | 3,00 |
| | pH=5,0 |
| Farbstoffe, Parfum, Konservierungsmittel | q.s. |
| Wasser | ad 100,00 |

Die Bestandteile der Ölphase werden vereinigt und homogenisiert, dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionstemperaturbereich des Systems wieder heraus gebracht).

**Beispiel 3**

O/W-Mikroemulsion

| | Gew.-% |
|---|---|
| Ceteareth-12 | 8,00 |
| Cetearylisononanoat | 10,00 |
| Mineralöl | 10,00 |
| Cetearylalkohol | 4,00 |
| Uvinul® T150 | 4,80 |
| $MgSO_4$ | 3,00 |
| Farbstoffe, Parfum, Konservierungsmittel | q.s. |
| Wasser | ad 100,00 |

Die unlösliche UV-Filtersubstanz wird im Mineralöl vorsuspendiert und mit der restlichen Ölphase vereinigt, worauf diese und homogenisiert und dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionstemperaturbereich des Systems wieder heraus gebracht) wird.

**Beispiel 4**

O/W-Mikroemulsion

| | Gew.-% |
|---|---|
| Ceteareth-12 | 12,00 |
| Cetearylisononanoat | 10,00 |
| Mineralöl | 10,00 |
| Cetearylalkohol | 6,00 |
| Eusolex ® 6300 | 2,00 |
| Parsol® 1789 | 0,50 |
| Uvinul® T150 | 4,80 |
| $MgSO_4$ | 3,00 |
| Farbstoffe, Parfum, Konservierungsmittel | q.s. |
| Wasser | ad 100,00 |

Die unlösliche UV-Filtersubstanz wird im Mineralöl vorsuspendiert und mit der restlichen Ölphase vereinigt, worauf diese und homogenisiert und dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionstemperaturbereich des Systems wieder heraus gebracht) wird.

**Beispiel 5**

O/W-Mikroemulsion

| | Gew.-% |
|---|---|
| Ceteareth-12 | 8,00 |
| Cetearylisononanoat | 10,00 |
| Cetearylalkohol | 4,00 |
| Eusolex® 232 | 5,00 |
| Uvinul® T150 | 1,00 |
| $MgSO_4$ | 3,00 |
| | pH=5,0 |
| Farbstoffe, Parfum, Konservierungsmittel | q.s. |
| Wasser | ad 100,00 |

Die Bestandteile der Ölphase werden vereinigt und homogenisiert, dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionstemperaturbereich des Systems wieder heraus gebracht).

**Beispiel 6**

O/W-Mikroemulsion

| | Gew.-% |
|---|---|
| Ceteareth-12 | 8,00 |
| Cetearylisononanoat | 10,00 |
| Mineralöl | 10,00 |
| Cetearylalkohol | 4,00 |
| Uvinul® T150 | 5,00 |
| $MgSO_4$ | 3,00 |
| Farbstoffe, Parfum, Konservierungsmittel | q.s. |
| Wasser | ad 100,00 |

Die unlösliche UV-Filtersubstanz wird im Mineralöl vorsuspendiert und mit der restlichen Ölphase vereinigt, worauf diese und homogenisiert und dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionstemperaturbereich des Systems wieder heraus gebracht) wird.

**Beispiel 7**

O/W/O-Emulsion

| | Gew.-% |
|---|---|
| Glycerylisostearat | 5,00 |
| Mineralöl | 25,00 |
| Stearinsäure | 2,00 |
| Uvinul® T150 | 2,00 |
| NaOH | ad pH 7,0 |
| Farbstoffe, Parfum, Konservierungsmittel | q.s. |
| Wasser | ad 100,00 |

Die unlösliche UV-Filtersubstanz wird in der Ölphase dispergiert. Wasser wird zugegeben und das System auf ca. 40° C erwärmt. NaOH wird zugegeben bis ein pH-Wert von 7 erreicht ist, hernach wird auf Raumtemperatur abgekühlt.

**Beispiel 8**

O/W/O-Emulsion

| | Gew.-% |
|---|---|
| Polyglyceryl-2-polyhydroxystearat | 7,00 |
| Cetearylisononaoat | 12,50 |
| Mineralöl | 12,50 |
| Stearinsäure | 2,00 |
| Uvinul® T150 | 2,00 |
| Parsol® 1789 | 2,00 |
| Eusolex® 232 | 2,00 |
| NaOH | ad pH 7,0 |
| Farbstoffe, Parfum, Konservierungsmittel | q.s. |
| Wasser | ad 100,00 |

Die unlösliche UV-Filtersubstanz wird im Mineralöl vorsuspendiert und mit der restlichen Ölphase vereinigt. Wasser wird zugegeben und das System auf ca. 40° C erwärmt. NaOH wird zugegeben bis ein pH-Wert von 7 erreicht ist, hernach wird auf Raumtemperatur abgekühlt.

**Beispiel 9**

O/W/O-Emulsion

| | Gew.-% |
|---|---|
| Polyglyceryl-2-polyhydroxystearat | 5,00 |
| Cetearylisononaoat | 12,50 |
| Mineralöl | 12,50 |
| Stearinsäure | 2,00 |
| Eusolex® 232 | 2,00 |
| Uvinul® T150 | 2,00 |
| Eusolex® 6300 | 2,00 |
| Parsol® 1789 | 1,00 |
| NaOH | ad pH 7,0 |
| Farbstoffe, Parfum, Konservierungsmittel | q.s. |
| Wasser | ad 100,00 |

Die unlösliche UV-Filtersubstanz wird in der Ölphase dispergiert. Wasser wird zugegeben und das System auf ca. 40° C erwärmt. NaOH wird zugegeben bis ein pH-Wert von 7 erreicht ist, hernach wird auf Raumtemperatur abgekühlt.

**Beispiel 10**

O/W-Makroemulsion

| | Gew.-% |
|---|---|
| Polyglyceryl-2-polyhydroxystearat | 2,00 |
| Cetearylisononaoat | 12,50 |
| Mineralöl | 12,50 |
| Stearinsäure | 2,00 |
| Uvinul®T150 | 2,00 |
| NaOH | ad pH 7,0 |
| Farbstoffe, Parfum, Konservierungsmittel | q.s. |
| Wasser | ad 100,00 |

Die unlösliche UV-Filtersubstanz wird im Mineralöl vorsuspendiert und mit der restlichen Ölphase vereinigt. Wasser wird zugegeben und das System auf ca. 40° C erwärmt. NaOH wird zugegeben bis ein pH-Wert von 7 erreicht ist, hernach wird auf Raumtemperatur abgekühlt.

**Patentansprüche**

**1.** O/W-Emulsionen, insbesondere O/W-Mikroemulsionen, oder O/W/O-Emulsionen oder O/W/O'-Emulsionen, enthaltend

- eine Wasserphase,
- gegebenenfalls übliche, in Wasser lösliche oder dispergierbare Substanzen,
- eine Ölphase,
  - in welcher mindestens eine in Ölkomponenten an sich schwerlösliche UV-Filtersubstanz,
    - beispielsweise gewählt aus der Gruppe 4,4',4"-(1,3,5-Triazin-2,4,6-triyltriimino)-tris-benzoesäure-tris(2-ethylhexylester), 2-Phenylbenzimidazol-5-sulfonsäure bzw. deren schwerlösliche Salze

  in suspendierter Form vorliegt,

- mindestens einen Emulgator (Emulgator A), gewählt aus der Gruppe der Emulgatoren mit folgenden Eigenschaften
  - ihre Lipophilie ist entweder abhängig vom pH-Wert, dergestalt daß durch Erhöhung oder Senkung des pH-Wertes die Lipophilie zunimmt oder abnimmt, wobei unerheblich ist, welche der beiden Möglichkeiten der Änderung der Lipophilie durch die Erhöhung bzw. Senkung des pH-Wertes bewirkt wird, und/oder
  - ihre Lipophilie ist abhängig von der Temperatur, dergestalt, daß die Lipophilie mit steigender Temperatur zunimmt und deren Hydrophilie mit sinkender Temperatur zunimmt,
  - ferner gegebenenfalls weitere in der Ölphase lösliche oder dispergierbare Substanzen, darunter bevorzugt solche, gewählt aus der Gruppe der nicht unter die Definition des Emulgators A fallende Emulgatoren, insbesondere solche, die vorwiegend als W/O-Emulgatoren wirken.

**2.** Verfahren zur Einarbeitung in Ölkomponenten an sich schwerlöslicher UV-Filtersubstanzen, in O/W-Emulsionen, O/W-Mikroemulsionen oder O/W/O-Emulsionen, dadurch gekennzeichnet, daß

- die Bestandteile einer Wasserphase,
- gegebenenfalls übliche, in Wasser lösliche oder dispergierbare Substanzen,
- die Bestandteile einer Ölphase
- mindestens ein Emulgator (Emulgator A), gewählt aus der Gruppe der Emulgatoren mit folgenden Eigenschaften
  - ihre Lipophilie ist entweder abhängig vorn pH-Wert, dergestalt daß durch Erhöhung oder Senkung des pH-Wertes die Lipophilie zunimmt oder abnimmt, wobei unerheblich ist, welche der beiden Möglichkeiten der Änderung der Lipophilie durch die Erhöhung bzw. Senkung des pH-Wertes bewirkt wird, und/oder
  - ihre Lipophilie ist abhängig von der Temperatur, dergestalt, daß die Lipophilie mit steigender Temperatur zunimmt und die Hydrophilie mit sinkender Temperatur zunimmt,
- in Ölkomponenten schwerlösliche UV-Filtersubstanzen, beispielsweise gewählt aus der Gruppe 4,4',4"-(1,3,5-Triazin-2,4,6-triyltriimino)-tris-benzoesäure-tris (2-ethylhexylester), 2-Phenylbenzimidazol-5-sulfonsäure bzw. deren Salze,
- ferner gegebenenfalls weitere in der Ölphase lösliche oder dispergierbare Substanzen, darunter bevorzugt solche, gewählt aus der Gruppe der nicht unter die Definition des Emulgators A fallende Emulgatoren, insbesondere solche, die vorwiegend als W/O-Emulgatoren wirken,

zusammengegeben werden und unter Agitation ein Gemisch gebildet wird, dergestalt, daß

- dieses Gemisch durch geeignete Wahl der Parameter, gewählt aus der Gruppe pH-Wert, Temperatur und der Konzentration bzw. Konzentrationen mindestens eines der gewählten Emulgatoren, in den Phaseninversionsbereich gebracht wird, in welchem sich W/O-Emulsionen in O/W-Emulsionen umwandeln,
- durch Variation mindestens eines Parameters, gewählt aus der Gruppe pH-Wert, Temperatur und der Konzentration bzw. Konzentrationen mindestens eines der gewählten Emulgatoren, die gebildete W/O-Emulsion aus dem Phaseninversionsbereich heraus gebracht wird, in welchem sich eine gebildete W/O-Emulsion in eine O/W-Emulsion umwandelt, wodurch eine O/W-Emulsion oder O/W-Mikroemulsion erzeugt wird
- gegebenenfalls durch geeignete Wahl der Rahmenbedingungen eine weitere Phaseninversion zur O/W/O-Emulsion eingeleitet wird,
- das Gemisch gegebenenfalls weiteren Aufbereitungsschritten, insbesondere einem oder mehreren Homogenisierungsschritten unterworfen wird.

3. Verfahren nach Anspruch 2 zur Herstellung von O/W/O-Emulsionen, dadurch gekennzeichnet, daß der Ölphasenanteil höher als etwa 15 Gew.-%, insbesondere höher als etwa 20 Gew.-%, bezogen auf das Gesamtgewicht der Zubereitung liegt, mehr als etwa 5 Gew.-%, insbesondere etwa 5 - 10 Gew.-% eines nicht unter die Definition des Emulgators A fallenden W/O-Emulgators vorliegen und/oder wenn die Ölphase einen niedrigen Anteil an polaren Ölen aufweist oder weitgehend frei von solchen Ölen ist.

4. Verfahren nach Anspruch 2 zur Herstellung von O/W-Mikroemulsionen, dadurch gekennzeichnet, daß der Ölphasenanteil unter etwa 20 Gew.-%, insbesondere unter etwa 15 Gew.-%, bezogen auf das Gesamtgewicht der Zubereitung liegt, weniger als etwa 5 Gew.-% eines zusätzlichen, nicht unter die Definition des Emulgators A fallenden W/O-Emulgators vorliegen und/oder wenn die Ölphase einen hohen Anteil an polaren Ölen aufweist.

5. Verfahren nach Anspruch 2 zur Herstellung von O/W-Makroemulsionen, dadurch gekennzeichnet, daß etwa 5 Gew.-% eines nicht unter die Definition des Emulgators A fallenden W/O-Emulgators, und mehr als etwa 20 Gew.-% einer polaren Ölphase vorliegen.

6. Verfahren nach Anspruch 2, dadurch gekennzeichnet, daß die in Ölkomponenten an sich schwerlösliche UV-Filtersubstanz oder -substanzen vor dem Emulgierungsvorgang in der Ölphase insbesondere vorteilhaft in einer unpolaren Ölkomponente bzw. einem Gemisch aus unpolaren Ölkomponenten, gegebenenfalls unter Erwärmen, dispergiert werden und die übrigen Schritte des Verfahrens nachgeschaltet werden.

7. OW-Makroemulsionen oder O/W-Mikroemulsionen oder O/W/O-Emulsionen oder O/W/O'-Emulsionen nach Anspruch 1, dadurch gekennzeichnet, daß der Emulgator A gewählt wird aus der Gruppe der polyethoxylierten ein- oder mehrbasigen Alkohole oder Fettsäuren, aus der Gruppe der Sorbitanester und Saccharoseester, insbesondere der verzweigten und unverzweigten Alkylester und Alkenylester mit Kohlenstoffketten von 4 - 24 Kohlenstoffatomen, bevorzugt Sorbitanstearat, Sorbitanoleat, Glycerylsorbitanstearat, Saccharosemonostearat, Saccharosemonolaurat, Saccharosepalmitat, der Monoglycerin-monocarbonsäure-monoester, der Di und Triglycerin-monocarbonsäure-monoester, ferner Triglyceryldiisostearat, Isostearyldiglycerylsuccinat, Diglycerylsesquiisostearat, Triglycerylpolyhydroxystearat, Cetearylisononanoat, Dicocoyl-pentaerythrityl-distearylcitrat, ferner die Methiconcopolyole, Cyclomethiconcopolyle, Alkylmethiconcopolyole, Laurylmethiconcopolyol, Cetyldimethiconcopolyol, sowie der Gruppe der verzweigten oder unverzweigten Alkylmonocarbonsäuren, Alkenylmonocarbonsäuren und Alkylendicarbonsäuren mit 4 bis 30 Kohlenstoffatomen, insbesondere Stearinsäure, Ölsäure, Bernsteinsäure, Hexansäure (Capronsäure), Heptansäure (Önanthsäure), Octansäure (Caprylsäure), Nonansäure (Pelargonsäure), Decansäure (Caprinsäure), Undecansäure, Undecensäure (Undecylensäure), Dodecansäure (Laurinsäure), Tridecansäure, Tetradecansäure (Myristinsäure), Pentadecansäure, Hexadecansäure (Palmitinsäure), Heptadecansäure (Margarinsäure), Octadecansäure (Stearinsäure), Isostearinsäure, Behensäure bzw. deren kosmetisch bzw. pharmazeutisch akzeptablen Salze, insbesondere der Alkali-, Ammonium, Monoalkylammonium, Dialkylammonium-, Trialkylammonium- und Tetraalkylammoniumsalze.

8. OW-Makroemulsionen oder O/W-Mikroemulsionen oder O/W/O-Emulsionen oder O/W/O'-Emulsionen nach Anspruch 1, dadurch gekennzeichnet, daß der Emulgator A oder die Emulgatoren A in Konzentrationen von 0,01 - 20 Gew.-%, bevorzugt 0,05 - 10 Gew.-%, besonders bevorzugt 0,1 - 5 Gew.-%, jeweils bezogen auf das Gesamtgewicht der Zusammensetzung, vorliegt oder vorliegen.

Fig. 1